# EUROPEAN PATENT SPECIFICATION

(11) **EP 2 787 333 B1**
(45) Date of publication and mention of the grant of the patent: **14.02.2018**
(21) Application number: 13001748.6
(22) Date of filing: 05.04.2013
(51) Int. Cl.: G01J 5/08, G01J 5/00, G01J 5/04, A61B 1/227, A61B 5/01, A61B 1/00

(54) **Ear inspection device and method of determining a condition of a subject's ear**
Ohrprüfungsvorrichtung und Verfahren zur Bestimmung des Zustandes des Ohrs eines Subjekts
Dispositif d'inspection d'oreille et procédé de détermination d'une condition de l'oreille d'un sujet

(43) Date of publication of application: 08.10.2014
(73) Proprietor: Helen of Troy Limited, St. Michael (BB)
(72) Inventor: Ruppersberg, J. Peter, 1807 Blonay (CH); Lepple-Wienhues, Albrecht, 25300 Pontarlier (FR)
(74) Representative: Graf von Stosch, Andreas

(56) References cited:
- EP-A1- 1 134 565
- US-A- 5 363 839
- US-A1- 2008 249 369
- US-A1- 2009 182 526
- US-A1- 2011 112 791
- US-A1- 2013 083 823

## Description

According to one aspect, the invention refers to an ear inspection device configured for being at least partially introduced into a subject's external ear canal for determining a condition of the subject's ear, such as temperature, in particular at the subject's eardrum, wherein the ear inspection device comprises an infrared sensor unit configured for detecting infrared radiation from the subject's ear.

Such ear inspection devices are known e.g. as radiation thermometers, infrared thermometers or ear thermometers. A corresponding thermometer is disclosed, for example in the US patent document US 6,898,457 B1 assigned to Braun GmbH. Such thermometers - not only used by physicians or other health care professionals but also by lay persons in the domestic field - have become more and more popular in recent years because the body core temperature can be determined very fast within only a few seconds with such a thermometer, and because measuring the temperature in the ear is generally more comfortable than measuring the temperature e.g. in the rectum with a classic mercury thermometer.

US 2013/083823 A1 discloses a thermometer, which comprises a probe adapted to be inserted into an orifice of the subject. An electromagnetic radiation sensor at the probe senses electromagnetic radiation within the orifice of the subject. The electromagnetic radiation sensor is configured to generate data indicative of both the temperature of the subject and one or more anatomical images of the subject.

US 2009/182526 A1 relates to an Infrared (IR) thermometer including an IR detector configured to provide an IR emission data representative of a temperature of an area of tissue. The IR thermometer also includes one or more secondary sensors configured to provide an IR thermometer positioning data.

Moreover, a portable clinical thermometer capable of providing visual images, is described in US 2011/112791 A1. The thermometer has a camera module mounted at a distal end, so as to take a body temperature and simultaneously take an image on a region that is suspected as having a disease.

However, even though the existing infrared sensor units are capable of measuring temperatures of the surface of an object relatively precisely (i.e. to an accuracy of about 1/10°C), the known ear thermometers, nevertheless, bear a risk of incorrect measurement of the subject's core temperature. The reason for this is that a free line of sight from the infrared sensor unit to the eardrum or tympanic membrane is mandatory for correctly measuring the subject's body core temperature. For example, if the device is oriented with respect to the subject's ear in such a way that the sensor is pointing to a surface of the exterior ear canal instead of to the eardrum, a too low temperature will be detected by the infrared sensor unit. The eardrum normally exhibits the highest temperature within the exterior ear canal, with the temperature of the eardrum substantially corresponding to the subject's body core temperature. For example, a normal temperature may be detected even when the subject has got high fever. Substantially the same is true if the ear canal is blocked by earwax, hair or dirt. In such a case, the infrared sensor unit will usually also detect a too low temperature (since there is no free line of sight to the eardrum). Obtaining unreliable or incorrect results for the body core temperature is critical, because a physician may thus make a wrong diagnosis and e.g. prescribe improper medicine to the subject.

It is therefore the object of the present invention to provide an ear inspection device of the kind described above but being able to overcome the previously mentioned drawbacks of the prior art.

This object is achieved by the subject-matter of claim 1. The subject-matters of the dependent claims refer to preferred embodiments. In particular, this object is achieved by an ear inspection device configured for being at least partially introduced into a subject's external ear canal for determining a condition of the subject's ear, such as temperature, in particular at the subject's eardrum, wherein the ear inspection device comprises an infrared sensor unit configured for detecting infrared radiation from the subject's ear, and wherein - according to the present invention - the ear inspection device further comprises
- an electronic imaging unit configured for capturing images based on radiation in the visible range from the subject's ear;
- a handle portion allowing a user to manipulate the ear inspection device during its application; and
- a head portion exhibiting a substantially tapering form extending along a longitudinal axis (A) of the head portion;
wherein the head portion has a proximal end adjacent to the handle portion and a smaller distal end configured to be introduced in the subject's external ear canal; wherein the electronic imaging unit is positioned at the distal end of the head portion;
wherein the electronic imaging unit and/or the infrared sensor unit is/are positioned radially offset from the longitudinal axis (A) of the head portion; and
wherein the ear inspection device further comprises a motion mechanism configured to allow rotational displacement of the electronic imaging unit and/or the infrared sensor unit around the longitudinal axis (A).

Providing the ear inspection device known in the art additionally with an electronic imaging unit allows for verifying whether there is a free line of sight from the infrared sensor unit to the subject's eardrum or not and, therefore, whether the results obtained by the infrared sensor unit are reliable or not. The risk of misdiagnosis can thus be effectively minimized when using an ear thermometer for rapidly and comfortably measuring a subject's core body temperature.

Preferably the electronic imaging unit captures images from the interior of the subject's external ear canal with a "main viewing direction" (or pointing direction) of the electronic imaging unit substantially corresponding to the one of the infrared sensor unit. Alternatively the main viewing direction of the electronic imaging unit may be angled with respect to the main viewing direction of the infrared sensor unit. In the latter case, both main viewing directions preferably intersect at a point at which the subject's eardrum is supposed to be when the ear inspection device is properly introduced into the subject's exterior ear canal. The images are preferably captured by the electronic imaging unit simultaneously or substantially immediately before measuring the temperature by the infrared sensor unit.

Verification of the free line of sight may be performed "manually" by the operator of the ear inspection device, e.g. if the captured image is shown on a display unit provided either integrally with the ear inspection device or separately thereof but operatively coupled thereto. Notably, the electronic imaging device may continuously capture images and provide these images to the operator in the form of a live video stream on the display unit. However, since such a "manual" verification can be prone to errors - especially when performed by lay persons - the verification may preferably be performed "automatically", e.g. by a logic unit capable of carrying out image recognition. With modern methods of image recognition it is possible to detect in a relatively reliable fashion whether the captured image shows the eardrum or not. Once at least one image has been captured by the electronic imaging unit, object recognition and unambiguous object identification (e.g. distinguishing objects, such as earwax, hair, and the eardrum) can be performed by determining brightness and/or color information of the pixels of the at least one captured image. Each pixel of the image obtained by the electronic imaging unit is characterized by a numerical value corresponding to the brightness of that pixel and - if the electronic imaging unit is a color camera - also by a numerical value corresponding to the color of that pixel. Different objects can thus be identified e.g. by their typical color and/or by brightness (when illuminated with a predetermined illumination source).

Usually, in pictures captured from the interior of a subject's ear, the eardrum is significantly darker than the wall of the exterior ear canal, usually showing a typical light reflex, when illuminated. In contrast to this, the captured image will show no particularly dark area when the exterior ear canal is blocked, e.g. with earwax. An optimum positioning or orientation of the ear inspection device with respect to the subject's ear may thus be obtained when the dark area (corresponding to the subject's eardrum) is substantially centered in the captured image.

The electronic imaging unit may be a video camera, preferably a wide angle video camera. The term "wide angle" in this context refers to angles of at least 80°, preferably of at least 110°, e.g. 120°. Such wide angle cameras allow detection of the subject's eardrum, even if the optical axis ("main viewing direction") of the camera is initially not directly centered to the eardrum. Once the eardrum has been detected in some region of the captured wide angle image, the operator of the ear inspection device may be informed, e.g. by some kind of guidance system, how to manipulate the position or orientation of the device with respect to the subject's ear so as to center the optical axis of the camera (and thus of the infrared sensor unit) to the eardrum. Preferably, a sequence of a plurality of images is captured by the electronic imaging unit, wherein only those data acquired by the infrared sensor unit may be taken into account that have been acquired with the optical axis of the camera substantially centered to the subject's eardrum. This allows reliably measuring the correct body core temperature. However, if it is not possible to detect the subject's eardrum in the image or images captured by the electronic imaging unit, a corresponding warning should be given to the operator of the ear inspection device. In such a case, a physician should be visited, e.g. because it may be necessary to clean the subject's external ear canal.

Notably, the term "infrared sensor unit" refers to all kinds of known and suitable infrared detectors as, for example, thermopiles, thermistors, bolometers, pyroelectric sensors, and semiconductor sensors.

Furthermore, the term "ear inspection device" is not limited to ear thermometers but refers to any kind of device for inspecting the subject's ear, in particular properties of the subject's eardrum. Thus, this term also refers to devices adapted for visual (otoscopic) inspection of the subject's ear, in particular the eardrum. Thus, the ear inspection device according to the present invention may be additionally or alternatively applied as video otoscope, wherein the electronic imaging device is preferably used to capture images of the interior of the subject's exterior ear canal. Advantageously, the electronic imaging unit is a color video camera so as to allow determination of the color of the eardrum and/or of the inner portion of the subject's exterior ear canal. Thus the degree of reddishness of any physiological objects in the ear canal can be determined (skin of the ear canal or of the eardrum). Determining the degree of reddishness of e.g. the eardrum may help the lay person to decide as to whether a physician should be visited or not, as it usually indicates inflammation of the eardrum. Inflammation of the eardrum may suggest e.g. an (bacterial/viral) infection. Any such more advanced or final disease diagnosis has to be carried out by the physician on the basis of other symptoms exhibited by the subject, which are observed by the physician or by the physician's further examination. Notably, disease diagnosis should not exclusively be derived from the output provided by the ear inspection device according to the invention.

When the ear inspection device according to the present invention is used as otoscope, there is a certain risk - especially if the operator is a lay person - that the images captured by the electronic imaging unit do not show the eardrum, but instead portions of the wall of the exterior ear canal and/or earwax, hair or dirt blocking the exterior ear canal and, thus, the free view on the eardrum. If the operator, e.g. a lay person, does not recognize that the image or images captured by the electronic imaging unit does/do not show the eardrum, he or she may conclude that there is probably no inflammation or infection of the eardrum and, therefore, that there is no need to consult a physician, even though this conclusion may be incorrect. To reduce this risk, the ear detection device according to the present invention preferably uses data measured by the infrared sensor unit to verify that the electronic imaging unit has a free line of sight to the subject's eardrum. As pointed out above, the main viewing direction of the electronic imaging device may substantially correspond to the one of the infrared sensor unit. Normally, the temperature at the surface of the eardrum (substantially corresponding to the subject's body core temperature) is higher than the temperature of the wall of the exterior ear canal and/or of earwax, hair or dirt in the exterior ear canal. Therefore, if the infrared sensor unit measures temperature values significantly (e.g. more than 2°C) below the normal body core temperature of a human being, this represents a strong hint that the main viewing direction of the infrared sensor unit (and, thus, also of the electronic imaging device) is not directed to the eardrum and/or that there is no free line of sight to the eardrum. A corresponding warning may be emitted to the operator of the device.

As also mentioned above, the ear inspection device may comprise some kind of a guidance system for instructing the operator how to manipulate the position or orientation of the device with respect to the subject's ear so that the main viewing direction of the infrared sensor unit (and, thus, preferably also of the electronic imaging device) is directed to the area with the highest temperature within the subject's exterior ear canal. Such a guidance system reduces the risk of capturing images not showing the subject's eardrum when the device according to the present invention is used as an otoscopic device.

For the above reasons, it is clear to those skilled in the art that the ear inspection device according to the present invention does not only provide the advantages of a known ear thermometer and of known (video) otoscopes, but that there additionally exists a strong synergetic effect of combining an infrared sensor unit and an electronic imaging unit within one device. That is, reliability of the data acquired by the infrared sensor unit or the electronic imaging unit can be significantly improved by the data acquired by the respective unit.

In order to benefit from this synergetic effect, the ear inspection device preferably further comprises a logic unit configured for receiving and processing signals (i.e. data) from the infrared sensor unit and the electronic imaging unit. The logic unit may be provided either integrally with the main portion of the ear inspection device or separately thereof - but operatively connected thereto. For example, the logic unit may form part of a remote device, such as a smart phone, having some kind of data connection with the remaining hardware of the ear inspection device.

It should be generally noted that the ear inspection device according to the present invention may comprise further features that are provided, for example, by modern digital photo cameras and known mobile phones. For example, the ear inspection device may comprise visual output means, such as a display, LEDs, etc., and/or acoustic output means, such as a loudspeaker, and/or a storage card slot for inserting a storage card to store the data acquired by the electronic imaging unit and/or the infrared sensor unit, and/or a cable connection port, such as an USB-port, and/or a wireless connection, such as Bluetooth®, WIFI®, and/or an energy supply, such as a battery.

Preferably, the logic unit is further configured for using the signals received from the electronic imaging unit and/or from the infrared sensor unit for verifying the correct positioning of the ear inspection device with respect to the subject's ear. As indicated above, such a verification process allows for enhancing reliability of the data acquired by the infrared sensor unit, or the electronic imaging unit by the data acquired by the respective unit. That is, either the image or images captured by the electronic imaging unit may be used to assure that the infrared sensor unit measures the temperature of the eardrum (i.e. the subject's body core temperature) or, *vice versa,* the temperature measured by the infrared sensor unit may be used to assure the correct "main viewing direction" and a free line of sight of the electronic imaging unit with respect to the subject's eardrum. Thus, it is possible to easily perform a plausibility check of the acquired data.

Additionally or alternatively, the logic unit may be further configured for determining - based on the received signals from the electronic imaging unit - whether the ear inspection device is positioned within the left or the right ear of the subject. To enable the device to distinguish between the left and the right ear has the following advantage: If an elevated temperature (i.e. a temperature above the normal body core temperature of a human being) is detected by the infrared sensor unit when the ear inspection device according to the present invention is introduced at least partially in one of the two exterior ear canals of the subject, this does not always allow to conclude that the subject has an elevated body core temperature, i.e. fever. Instead, the measured elevated temperature may result from a local inflammation of the eardrum of the ear into which the device has been introduced. Local inflammations also lead to a raise in temperature at the site of inflammation. To distinguish between these two cases, i.e. fever vs. local inflammation, it is advantageous to subsequently carry out the temperature measurement at both (i.e. left and right) ears of the subject. If the subject has got fever, the temperatures detected in both ears are supposed to be substantially the same, whereas, in the case that there is a local inflammation of an eardrum, the temperatures detected in both ears are supposed to differ significantly. Notably, it is rather unlikely that both eardrums are simultaneously inflamed - in particular inflamed to the same degree. In order to avoid any mistakes when subsequently performing several measurements of the temperature, it is advantageous if the device can automatically determine whether the temperature was measured within the left or the right ear of the subject. Only if at least one temperature signal from the left ear and at least one temperature signal from the right ear are available, the ear inspection device may compare the measured temperature signals so as to determine - and preferably inform the operator of the device - as to whether the subject has got fever and/or a local infection. If only measured temperature values from one ear, either the left or the right ear, are available, the device may inform the operator to carry out the measurement at the respective other ear of the subject.

Since there are specific differences in the aspects of the left exterior ear canal and the right exterior ear canal, modern image recognition methods are capable of relatively reliably distinguishing between images captured from the interior of the left ear and images captured from the interior of the right ear.

Notably, the images captured by the electronic imaging unit may additionally be used to detect a local inflammation at the interior of a subject's ear by determining the degree of reddishness in the captured image.

In order to improve the identification of objects in the subject's ear, the logic unit is preferably further configured for discriminating different objects in the subject's ear, such as earwax, hair and the eardrum, by comparing their appearance in at least two images captured by the electronic imaging unit from different positions within the ear canal and/or with illumination from different positions within the ear canal.

The electronic imaging unit and preferably at least one light source may be introduced into an exterior ear canal of a subject; the electronic imaging unit may then be used to capture at least two images from different positions within the ear canal and/or with illumination from different positions within the ear canal; and the at least two captured images may be compared with each other to identify objects shown in the images.

In order to capture at least two images from different positions within the subject's exterior ear canal, the electronic imaging unit may be relocated when placed in the subject's ear canal and/or at least one further electronic imaging unit may be provided, wherein the two or more electronic imaging units are positioned at different sites in the ear canal. Alternatively or additionally, there may be provided at least one illumination unit which is adapted to illuminate objects within the ear canal from different positions (e.g. from two or more positions). Preferably, a combination of both approaches is realized by the inventive device, which allows capturing images from different positions under differing illumination conditions. Such a mode of action allows for reliable identification of distinct objects (e.g. the eardrum, particles of earwax, hair, etc. in the subject's ear), as will be described in more detail below. Thereby, the risk of image misinterpretation and failure in object recognition is significantly reduced.

If at least two images are captured from different positions within the ear canal, different objects, such as the eardrum and other objects, are discriminated by comparing their positions as provided in the at least two images. That is, it is possible to determine the distance of various objects in the ear canal with respect to the electronic imaging unit according to the fundamental principle of stereoscopic viewing, also known as "parallax". Parallax is a displacement or difference in the apparent position of an object viewed along two different lines of sight, and is measured by the angle or semi-angle of inclination between those two lines. For example, a person closing only his left eye sees objects being relatively close at a position other than by closing only his right eye. However, the person will see relatively remote objects substantially at the same position. The human brain is thus able to determine the distance from the observer to the objects as a result of the parallax phenomenon. The same approach may be realized by the logic unit of the ear inspection device according to the inventive method when capturing images from different positions within the ear canal. Since the electronic imaging unit will not and cannot be introduced too deeply into the subject's ear canal for not causing harm to the eardrum, the eardrum, as the membrane (object) terminating the ear canal, is relatively remote with respect to the electronic imaging unit, whereas other objects in the ear canal positioned more proximal to the electronic imaging unit are recognized as being less remote from the imaging unit as reference point. Thus, by the inventive method, e.g. the eardrum can be readily distinguished from other objects located more proximal in the ear canal.

Alternatively or additionally, different objects, such as earwax, hair, and the eardrum, within the subject's ear canal may be discriminated by comparing their appearance as depicted by at least two images captured under illumination from different positions (for each single image) within the ear canal. If an object positioned relatively closely to the electronic imaging unit, such as earwax, is illuminated from different positions within the ear canal (by e.g. two or more distinct light sources or by e.g. one single light source which can be repositioned), the appearance of such an object will significantly differ in the at least two captured images. Usually, the position of the sources of illumination is chosen such that they are still positioned closely to the electronic imaging unit. In contrast thereto, an object positioned relatively remote from the electronic imaging unit, such as the eardrum, will typically not change its appearance in the at least two captured images by such illumination from different positions.

If e.g. massive earwax blocking the subject's external ear canal has been detected by the logic unit as described above, the operator of the device may be informed correspondingly. In particular, he or she may be informed that a reliable temperature measurement is not possible (since there is no free line of sight between the infrared sensor unit and the eardrum). The subject may then go to see a doctor for having his or her ear professionally cleaned. Additionally and/or alternatively, the ear inspection device according to the present invention may comprise or may be combined with a flushing and/or suction unit in order to remove earwax based on the results of the earwax detection without the need to see the doctor. Corresponding flushing and/or suction units for cleaning ears are known in the art.

The infrared sensor unit of the ear inspection device according to the present invention may comprise a plurality of infrared sensor elements for detecting infrared radiation from different regions of the ear. Such an infrared sensor is disclosed in the previously mentioned US patent document US 6,898,457 B1 assigned to Braun GmbH. Preferably, only the temperature signal of the particular sensor element is used which supplies the peak temperature value by comparison with the remaining sensor elements. This peak temperature value most likely represents the temperature at the subject's eardrum since the eardrum usually exhibits the highest temperature within the subject's exterior ear canal.

More preferably, the infrared sensor unit of the ear inspection device according to the present invention may be formed by an infrared camera configured for capturing images based on radiation in the infrared range from the subject's ear. This allows for obtaining a two-dimensional image of the temperature distribution in the area observed by the infrared camera.

The electronic imaging unit and/or the infrared camera may be a wafer-level camera of a substantially flat configuration. Such wafer-level cameras may have dimensions of less than 3mm x 3mm, preferably less than 2mm x 2mm, even more preferably of about 1 mm x 1 mm or even less than 1 mm x 1 mm. Wafer-level cameras refer to a relatively new technology. They can be produced small in size with only about 3 microns per pixel. Therefore, wafer-level imaging technology allows obtaining images (of the temperature distribution and/or of light in the visual range) of "sufficient" resolution of the eardrum, e.g. images of 250 pixels x 250 pixels, with a footprint of the camera (including a lens) of only about 1 mm x 1 mm or even smaller.

Notably, existing wafer-level cameras usually comprise photo-sensitive elements already being sensitive to light in the infrared range (and not only to light in the visible range). However, the photo-sensitive elements of the existing wafer-level cameras are covered by filters. Usually one image pixel of the wafer-level camera is defined by four different photo-sensitive elements, one covered by a filter for allowing only red light to pass, one covered by a filter for allowing only green light to pass, one covered by a filter for allowing only blue light to pass, and a final one for determining the brightness. However, for a manufacturer of wafer-level cameras it is relatively easy to obtain a wafer-level camera sensitive to light in the infrared range by merely replacing at least one of the filters of the four photo-sensitive elements defining a pixel by a filter for allowing only infrared light to pass.

In order to reduce manufacturing costs of the inventive ear inspection device, the infrared sensor unit may be formed integrally with the electronic imaging unit. For example, the filters for allowing only blue light to pass may be replaced by filters for allowing only infrared light to pass. Even though such a design change of an existing waver-level camera does not allow for obtaining true-color images in the visible range from the subject's ear canal, for the purpose of the present device it may suffice to obtain images (in the visible range) only based on red and green colors. Alternatively, one may think about applying the four photo-sensitive elements of e.g. every second pixel of an existing waver-level with filters for allowing only infrared light to pass. Thus, the resolution (of images of visible light) of the waver-level camera would be reduced, e.g. to half. Moreover, it would be possible to further redesign an existing waver-level camera by providing five photo-sensitive elements per each image pixel of the camera, namely an additional photo-sensitive element having a filter for allowing only infrared light to pass.

Providing the infrared sensor unit integrally with the electronic imaging unit (i.e. providing both on the same chip) exhibits the further advantage that the main viewing direction of the infrared sensor unit will (automatically) coincide with the main viewing direction of the electronic imaging unit.

Advantageously, the ear inspection device of the present invention further comprises a fluid sensor unit adapted to detect fluid in the subject's ear. A fluid sensor unit represents a sensor unit for inspecting the mobility of the tympanic membrane. The detection of fluid in the ear represents another factor in the diagnosis of acute otitis media or severe ear infection. If fluid is accumulated behind the eardrum, the latter cannot vibrate as freely as normally when subjected to pressure or acoustic waves. Therefore, the waves reflected from the eardrum will hardly be absorbed and/or attenuated by the eardrum. This can be determined e.g. by using an acoustic transducer and a microphone according to a technique known as "acoustic reflectance". This technique is described in detail in US patent document US 5,868,682 B1. However, the technique of the fluid sensor unit may be based on any known technique, such as - but not limited to - acoustic reflectance, tympanometry and otoacoustic emissions.

For example, the fluid sensor unit may comprise pressurization means configured for applying a varying pressure within the subject's external ear canal, and optical means configured for inspecting the mobility of the subject's tympanic membrane when exposed to the varying pressure. This technique is also known as "pneumatic otoscopy", wherein this technique traditionally does not apply an electronic imaging unit but conventional optical means for visual inspection. The pressure is preferably applied by (compressed) air, wherein an air-tight chamber is formed by the subject's external ear canal and the corresponding device. Preferably the optical means comprises the electronic imaging unit or is substantially defined by the electronic imaging unit. Using the electronic imaging unit of the ear inspection unit according to the present invention for determining the mobility of the eardrum when subjected to varying pressure, allows for omitting the usually applied optical means for visual inspection (such as multiple lenses), thereby achieving another synergetic effect.

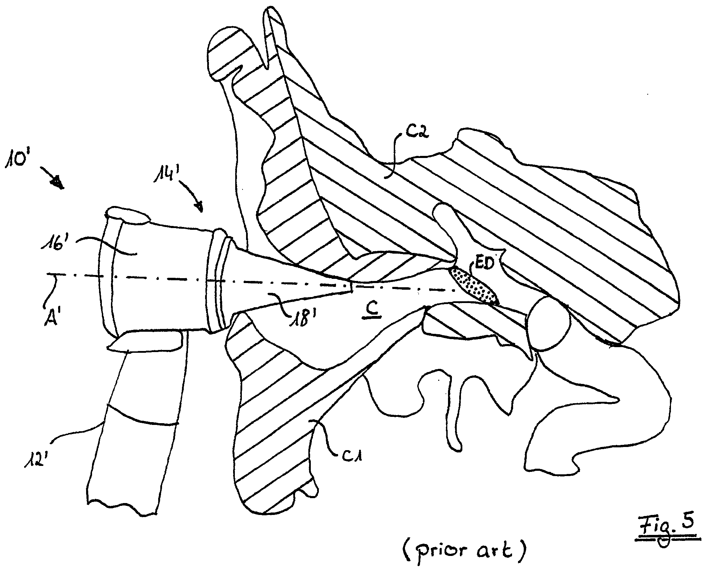

Another problem with otoscopes known in the art is that the ear has to be significantly deformed - which is uncomfortable or even causes pain to the subject - in order to have a direct view onto the eardrum. Furthermore, there is a risk of introducing the otoscope too far into the subject's interior ear canal thereby causing pain and injuries to the ear canal, and in particular to the eardrum. In order to better illustrate these problems reference to figure 5 is made in the following.

Figure 5 shows a typical otoscope 10' as used for decades in otoscopy. The otoscope 10' comprises a handle portion 12' allowing the user to manipulate the otoscope during its application. The term "to manipulate" in this context refers to different kinds of manipulation, such as - but not limited to - holding the otoscope, aligning the otoscope with respect to the subject's ear, and turning on or off a light. The otoscope 10' further comprises a head portion 14' connected to the handle portion 12'. The head portion 14' exhibits a substantially tapering form - usually a conical form - extending along a longitudinal axis A' of the head portion 14'. The head portion 14' is substantially comprised of an empty funnel, wherein the tip of the funnel typically has a diameter of 3mm. Furthermore, the head portion 14' has a proximal end 16' adjacent to the handle portion 12' and a smaller distal end 18' configured to be introduced in an ear canal C of a subject's outer ear. The term "end" in this context does not mean a single point but rather refers to a region or section of the head portion 14', wherein the proximal end 16' is located opposite to the distal end 18' with respect to the longitudinal axis A'. The ear canal C is partly surrounded by soft connective tissue C1 hand - further down towards the middle ear - partly by hard bone C2.

The working principle of the known otoscope is typically to observe and simultaneously illuminate the subject's eardrum ED through the empty funnel with the 3mm tip pushed deeply into the ear canal C. Normally, the eardrum ED is not visible from outside the ear, due to the natural curvature of the ear canal C. In order to overcome the natural curvature of the ear canal C, the skilled physician has to carefully pull the outer ear upward and to the back while carefully pushing the tip of the funnel as deeply as necessary to observe the eardrum. The ear canal C has to be deformed in such a way that the physician has a free view onto the eardrum ED along the optical axis of the otoscope 10', wherein the optical axis corresponds to the longitudinal axis A' of the head portion 14'. The optics of an otoscope is situated only at the wider end of the funnel at its proximal end 16' and essentially consists of a lamp and a lens (not shown) to magnify the image of the eardrum ED.

The otoscopy procedure thus needs manual skills and significant training to carefully push the funnel into the ear canal C while looking inside and manipulating the curvature of the ear canal C by pulling the ear. For example, it is very important for the trained physician to brace the hand holding the otoscope against the subject's head to avoid injury to the ear canal C by placing the index finger or little finger against the head. In particular in the case of young children - where the inner part of the ear canal is relatively short and sudden head movement during the examination may occur - there is a risk of penetration of the very sensitive ear canal skin or even of the eardrum ED. Besides pain and handicapped hearing, such an injury may even induce cardiovascular complications through a vagal overstimulation and therefore has to be avoided by all means.

Moreover, especially in an inflamed ear, the mechanical manipulation of "straightening" the ear canal C typically causes considerable discomfort or even pain, rendering the examination of an infant even more difficult.

For the above reasons, reliable and secure handling an otoscope of the art is currently subject to only well trained physicians and not amenable to the larger community of practitioners. A study recently published in the US as a result of a survey has shown that even physicians often fail to (correctly) determine the status of e.g. the subject's eardrum or fail to correctly interpret the image provided by the otoscope (i.e. correct and meaningful object recognition). Such failures result in misinterpretation of the status of the inner ear canal or the eardrum. As a consequence, e.g. over-medication with antibiotics for treating supposed inflammations of the eardrum occurs, because physicians tend to err on the side of caution, or meaningless image interpretation occurs.

In view of these drawbacks of the known otoscopes, the ear inspection device according to the present invention further comprises a handle portion allowing a user to manipulate the ear inspection device during its application, and a head portion exhibiting a substantially tapering form extending along a longitudinal axis of the head portion, wherein the head portion has a proximal end adjacent to the handle portion and a smaller distal end configured to be introduced in the subject's external ear canal, and wherein the electronic imaging unit is positioned at the distal end of the head portion.

By providing the preferably relatively small electronic imaging unit at the distal end of the head portion it becomes possible to "see" the subject's eardrum without the need to deform the subject's ear canal, or at least without having to deform the ear canal to such an extent as with the above described conventional otoscope. The reason for this is that there is no need for the "viewing direction" (optical axis) of the electronic imaging unit to correspond to the longitudinal axis of the head portion of the otoscope. Instead, the optical axis of the electronic imaging unit may be arranged at an angle with respect to the longitudinal axis, allowing the device to "look around the corner". An additional or alternative reason is that the field of vision of an electronic imaging unit provided at the distal end of the head portion can be much greater than the field of vision achievable with the relatively acute empty funnel of the otoscope according to the prior art.

Furthermore, in contrast to conventional otoscopes, the distal end of the head portion of the ear inspection device according to the present invention does not need to have a conical shape with a relatively thin open funnel, which shape bears the risk of introducing the distal end of the head portion too far into the ear canal, so as to cause serious injuries to the subject. Instead, the outer shape of the distal end of the head portion can be designed in such a way that it is practically impossible to introduce it too far into the ear canal. Thus, the otoscope according to the present invention can be securely and reliably operated even by lay persons without the risk of causing injuries to the subject.

In other words, the ear inspection device of the present invention allows for domestical inspection of a subject's ear by lay persons and medical doctors without extensive otoscopy training and without any - or at least with a significantly reduced - risk of causing injuries to the subject.

The electronic imaging unit or the infrared sensor unit may be positioned substantially centrically with respect to the longitudinal axis of the head portion. If the electronic imaging unit or the infrared sensor unit is positioned on the longitudinal axis of the head portion, the substantially flat electronic imaging unit or the infrared sensor unit is preferably inclined with respect of the longitudinal axis of the head portion, so that the optical axis (or the "main viewing direction") of the electronic imaging unit or the infrared sensor unit is angled with respect to the longitudinal axis of the head portion, allowing the ear inspection device to "look around the corner". Consequently, the ear inspection device according to the present invention does not have to be introduced as deeply into the subject's ear as in a conventional device of the prior art.

The electronic imaging unit and/or the infrared sensor unit is/are positioned radially offset from the longitudinal axis of the head portion. Such a configuration also allows obtaining a free view onto the eardrum without having to introduce the electronic imaging unit and/or the infrared sensor unit as deeply as it would be necessary if the electronic imaging unit and/or the infrared sensor unit were placed just centrally on the longitudinal axis of the head portion. The offset may be at least 1 mm, preferably at least 2mm, more preferably at least 3mm from the longitudinal axis.

The head portion is preferably shaped in such a way that its distal end comprising the electronic imaging unit, and preferably also the infrared sensor unit, can be introduced only as deeply into the subject's ear canal as not to touch the eardrum. The ear canal of the subject's outer ear is limited by the eardrum. Notably, the ear canal of the subject's outer ear comprises an outer part which refers to a portion of the subject's outer ear (i.e. the subject's external auditory canal) that is surrounded by soft connective tissue and that usually comprises hair and earwax. The outer part comprises approximately the outer half of the ear canal of the subject's outer ear. Furthermore, the ear canal of the subject's outer ear also comprises an inner part which refers to a portion of the subject's outer ear (i.e. the subject's external auditory canal) that is surrounded by hard skull bone and that is usually free from any hair and earwax. This portion extends from the proximal end of the outer part of the ear canal of the subject's outer ear to the eardrum. The inner part of the ear canal is very sensitive to pain in case of mechanical friction. Injuring the inner part of the ear canal even bears the risk of cardiovascular complications through vagal overstimulation.

Preferably, a tip portion of the distal end can be introduced into the ear canal of the subject's outer ear no further than a few millimeters, preferably of at least 3mm, from the eardrum.

As already mentioned above, the tapering head portion of the ear inspection device according to the present invention may be shaped with a blunt, rounded tip end, as compared to conventionally known devices, thereby reducing the risk of introducing injury or discomfort to the subject. Thus, the device can be securely handled by lay persons. The otoscope according to the present invention, nevertheless, allows detecting the eardrum, since the electronic imaging unit and/or the infrared sensor unit is/are provided at the distal end of the head portion.

Preferably, the distal end of the head portion is provided with a round and smooth shape. Moreover, the distal end may be made from a relatively soft material, such as silicone, or it may comprise an outer surface made of such a soft material. Furthermore, the longitudinal force upon introduction into the ear canal can be limited by a telescoping mechanism or the use of an elastic element.

When introducing the tip end of the head portion no deeper into the ear canal than to the border between the outer part and the inner part of the outer ear canal of the subject's outer ear, there is the risk that artifacts, such as earwax, hair and other kind of dirt from the outer part of the outer ear canal, obstruct the view of the small electronic imaging unit onto the eardrum. Therefore, as already mentioned above, it is advantageous to take several images from different positions within the ear canal. For doing so, the ear inspection device according to the present invention may comprise more than one electronic imaging unit at the distal end of its head portion, e.g. two imaging units, located at different positions on the head portion.

The ear inspection device according to the present invention further comprises a motion mechanism configured to allow displacement of the electronic imaging unit and/or the infrared sensor unit relative to the handle portion. With such a motion mechanism, it is possible to capture a plurality of images or to acquire a plurality of temperature signals from different positions by one single electronic imaging unit and/or one single infrared sensor unit within the subject's ear canal, thereby avoiding the need for two or more electronic imaging units and/or infrared sensor units. If, for example, a hair - at least partially - obstructs the view of the electronic imaging unit and/or the infrared sensor unit at a certain position within the ear canal onto the eardrum, the electronic imaging unit and/or the infrared sensor unit may have a free view onto the eardrum at another position in the ear canal or may at least have a free view onto the part of the eardrum that was partially obstructed by the one hair before.

Moreover, providing such a motion mechanism also allows for automatic identification of different objects in the subject's ear according to the principle of stereoscopic viewing, as explained in more detail above.

The motion mechanism is configured to allow at least partial rotation of the electronic imaging unit and/or the infrared sensor unit about an axis of rotation. The axis of rotation corresponds to the longitudinal axis of the head portion. By displacing the electronic imaging unit along a predefined motion path, it is possible to automatically calculate the distance between the electronic imaging unit and the detected objects, as described above. In view of the typical size of the artifacts found in the ear canal, such as hair and earwax particles, the motion mechanism preferably allows for displacement of the electronic imaging unit and/or the infrared sensor unit of at least 1 mm within the subject's ear canal. A rotation of at least 90°, more preferably of at least 120°, even more preferably of 180° or even more degrees around the axis may be realized. Preferably, the motion mechanism allows for rotation in both directions, i.e. clockwise and counter-clockwise. The motion mechanism may also allow for rotational displacement about more than one axis. The motion mechanism may comprise at least one motor and one or more gears and/or bearings. The electronic imaging unit and/or the infrared sensor unit may be connected to a flexible cable, e.g. a flexible ribbon cable, to allow for such a movement.

Preferably, the electronic imaging unit and/or the infrared sensor unit is/are tilted against the axis of rotation so as to be continuously directed to a predetermined point on the axis of rotation, the predetermined point having a fixed distance to the electronic imaging unit and/or the infrared sensor unit. In view of the typical length of the inner part of the outer ear canal of the subject's outer ear, the distance may be between 3mm and 20mm. Thus, the optical axis ("viewing direction") of the electronic imaging unit and/or the infrared sensor unit is optimized for centering on the eardrum, which usually represents the object of primary interest within the subject's ear.

For hygienic reasons, the ear inspection device preferably further comprises an at least partially transparent (preferably to both, visual and infrared light) probe cover configured to be put over the head portion. The probe cover may be made from a plastic material, preferably from a transparent plastic material. Such a probe cover may be designed as a single-use product that can be produced in large numbers with low costs. The probe cover shall be transparent, at least at the locations where it covers the electronic imaging unit and/or the infrared sensor unit, so as to allow the electronic imaging unit and/or the infrared sensor unit to have a clear view onto the eardrum. The probe cover also inhibits contamination of the head portion of the ear inspection device, in particular when introducing the head portion into the subject's ear canal.

Preferably, the probe cover is adapted to be fixed to at least one section of either the head portion and/or the handle portion in such a way that the probe cover does not move relative to the handle portion during displacement of the electronic imaging unit and/or the infrared sensor unit by the motion mechanism. Otherwise, artifacts, such as earwax particles, adhering to the probe cover will obstruct a free view onto the eardrum, even if the electronic imaging unit and/or the infrared sensor unit is/are displaced by the motion mechanism.

The ear inspection device may further comprise a probe cover moving mechanism adapted to move at least a portion of the probe cover with respect to the electronic imaging unit and/or infrared sensor unit. Thus, artifacts, such as earwax particles, adhering to the probe cover and obstructing the view of the electronic imaging unit and/or infrared sensor unit onto the eardrum can be moved away from the electronic imaging unit and/or infrared sensor unit by the probe cover moving mechanism.

Preferably, the probe cover is designed in a way that allows unfolding or peeling off portions of the probe cover in order to move portions of the probe cover contaminated e.g. with earwax, away from the electronic imaging unit and/or infrared sensor unit. The otoscope preferably contains mechanical means to move the probe cover against the electronic imaging unit and/or infrared sensor unit or *vice versa.*

In order to illuminate the subject's ear canal and eardrum for taking images thereof, the ear inspection device may further comprise at least one light source also positioned at the distal end of the head portion. The term "light source" is understood to apply to any source capable of emitting photons.

Since geometrical restrictions limit the space at the distal end of the head portion, the light source is preferably formed by the distal end of a light guide. For example, the light guide may exhibit a diameter of less than 1 mm, preferably of less than 0.5mm, more preferably of about 0.2mm. The light guide may be connected to an LED located remote from the distal end of the head portion. The light guide may be e.g. a nylon light guide, preferably having a diameter of only about 0.2mm to 1 mm. Alternatively, a light source may be formed e.g. by a small light-emitting diode that is placed directly at the distal end of the head portion. However, attention should be paid that heat stemming from the light-emitting diode does not adversely affect the measurements of the infrared sensor unit. Therefore, it is preferred to locate the light-emitting diode remote from the infrared sensor element. For example, the infrared sensor unit may be located at the tip end of the head portion of the ear inspection device, whereas the light-emitting diode is located remote thereof having a light guide leading to the tip end of the head portion. Light guides usually filter out any infrared radiation, only emitting "cold" light.

It should be further mentioned that the infrared sensor unit may comprise some kind of heating and controlling mechanism so as to heat the infrared sensor unit to a predefined temperature before carrying out any measurements therewith. Thus, the accuracy of the measurement can be improved.

It is advantageous if the ear inspection device comprises a plurality of light sources at the distal end of the head portion, preferably with each light source being controllable separately. By illuminating objects in the subject's ear canal from different positions, e.g. by sequentially switching on and off the individual light sources, it is possible - as mentioned above - to distinguish different objects in the ear, without necessarily having to displace the electronic imaging unit by a motion mechanism within the ear canal.

Additionally or alternatively, the at least one light source may be controllable in view of the color, so that it is possible to change the color of the light emitted by the light source. For example, red color may be preferred to recognize an inflamed eardrum, wherein green color may be preferred to recognize earwax.

Like the electronic imaging unit and/or the infrared sensor unit, the at least one light source is preferably positioned radially offset from the longitudinal axis of the head portion. Such a configuration allows illumination of the eardrum without the need to introduce the light source as deeply into the ear canal as it would be necessary if the light source were placed centrally on the longitudinal axis of the head portion. The offset may be at least 1 mm, preferably at least 2mm, more preferably at least 3mm from the longitudinal axis.

Preferably, the at least one light source is arranged so as to maintain a predetermined distance with respect to the electronic imaging unit, even when the electronic imaging unit is displaced by the motion mechanism. Such a configuration is advantageous because the predetermined distal relationship between the at least one light source and the electronic imaging unit allows for improved (automatic) image analysis. If a motion mechanism is provided, the motion mechanism preferably also displaces the at least one light source. If the light source is provided in the form of a light guide, the light guide should be sufficiently flexible to allow for such a displacement of the at least one light source.

According to another aspect, the present invention also refers to a corresponding method of determining a condition of a subject's ear, such as temperature, in particular at the subject's eardrum, wherein the method comprises the following steps:
- introducing the ear inspection device according to the present invention as described above, at least partially into the subject's external ear canal, the ear inspection device comprising an infrared sensor unit and an electronic imaging unit;
- detecting infrared radiation from the subject's ear using the infrared sensor; and
- capturing at least one image based on radiation in the visible range from the subject's ear using the electronic imaging unit.

As described above, there exists a strong synergetic effect of detecting infrared radiation from the subject's ear combined with capturing at least one image based on radiation in the visible range from the subject's ear. That is, reliability and accuracy of the data acquired by the infrared sensor unit or the electronic imaging unit can be significantly improved by the data acquired by the respective unit.

The method according to the present invention may further comprise at least one of the following steps:
- verifying appropriate positioning of the ear inspection device with respect to the subject's ear based on the detected infrared radiation and/or the at least one captured image;
- determining whether the ear inspection device is positioned within the left or the right ear of the subject based on the at least one captured image; and
- discriminating different objects in the subject's ear, such as earwax, hair and the eardrum, by comparing their appearance in at least two images captured by the electronic imaging unit from different positions within the ear canal and/or with illumination from different positions within the ear canal.

Two exemplary embodiments of an ear inspection device according to the present invention will be described in more detail in the following with respect to the drawings, wherein:
- figure 1: schematically shows a cross-sectional view of a head portion and of a part of a handle portion of a first embodiment of an ear inspection device according to the present invention;
- figure 2: shows an enlarged view of a plate covering a bore provided in the head portion illustrated in figure 1;
- figure 3: schematically shows a cross-sectional view of a head portion and of a part of a handle portion of a second embodiment of an ear inspection device according to the present invention;
- figure 4: shows an enlarged view of a plate covering a bore provided in the head portion illustrated in figure 3; and
- figure 5: shows an otoscope of the prior art, with its head portion partially introduced into the subject's ear canal.

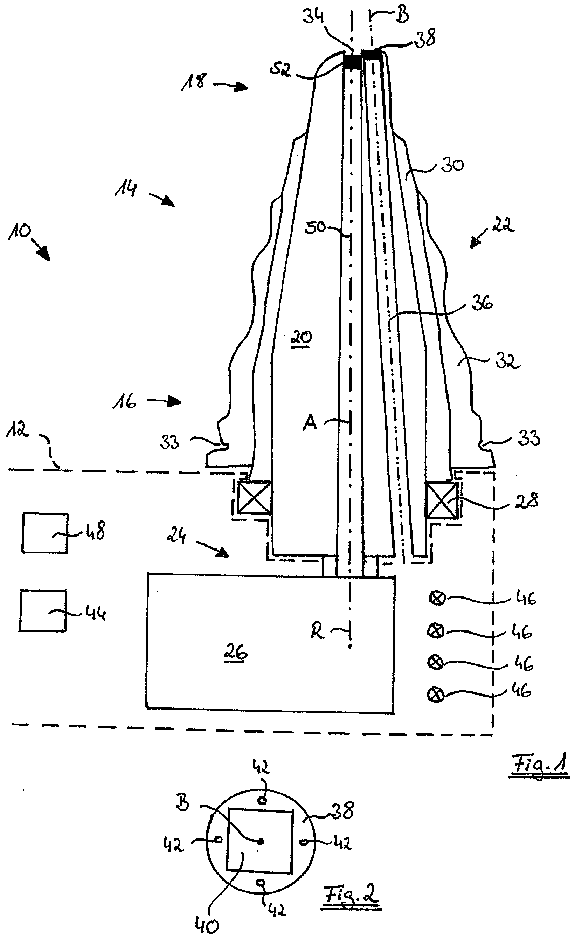

Figure 1 schematically shows a cross-sectional view of a head portion 14 and a part of a handle portion 12 (only shown in phantom lines) of a first embodiment of an ear inspection device 10 according to the present invention. As can be seen from figure 1, the head portion 14 has a substantially tapering form extending along a longitudinal axis A of the head portion 14. The head portion 14 comprises a relatively large proximal end 16 adjacent to the handle portion 12 and a smaller distal end 18. The distal end 18 of the head portion 14 is adapted to be introduced into a subject's ear canal.

Furthermore, the head portion 14 comprises a rotatable, radial inner portion 20 and a fixed, radial exterior portion 22. The rotatable portion 20 is rotatable about an axis of rotation R which - in the shown exemplary embodiment - corresponds to the longitudinal axis A of the head portion 14. A motion mechanism 24 comprising a servo motor 26 is positioned within the handle portion 12 and is coupled to the rotatable portion 20 of the head portion 14, so as to rotate the rotatable portion 20 about its axis of rotation R relative to the fixed portion 22 of the head portion and relative to the handle portion 12 of the ear inspection device 10. The rotatable portion 20 is supported by a radial bearing 28 (also only schematically shown).

In the shown exemplary embodiment, the exterior portion 22 of the head portion 14 comprises a support structure 30 providing the required stability of the head portion 14. The support structure is at least partially covered by an outer cladding 32 formed from a relatively soft material, such as silicone. The cladding 32 makes the introduction of the distal end 18 of the head portion 14 into his ear canal more comfortable for the subject. The cladding may comprise a circular slot-like recess 33 adapted to engage with a complementarily formed circular tongue of a (not shown) probe cover. The probe cover may be formed from a plastic material and may be adapted to be put over the head portion 14. Preferably, the probe cover is formed from a transparent material, preferably transparent to both, visible and infrared light. Its wall may be relatively thin, thereby making the probe cover relatively flexible. At least a portion of the probe cover covering the distal end 18 of the head portion 14 should be transparent, so as to allow an electronic imaging unit and an infrared sensor unit (described in the following) which are located at the distal end 18 of the head portion 14 to have a free view through the probe cover. For hygienic reasons, the probe cover is preferably designed as a single-use product. The probe cover also reliably inhibits contamination of the distal end 18 comprising the electronic imaging unit and the infrared sensor unit. Without such a probe cover there is a high risk that e.g. earwax particles may adhere to the electronic imaging unit and/or the infrared sensor unit (thereby deteriorating the detection quality thereof) when introducing the distal end 18 into the outer part of the subject's exterior ear canal.

The head portion 14 comprises a distal end point 34 which, in the shown exemplary embodiment, is located substantially on the longitudinal axis A of the head portion 14. However, the head portion 14 may alternatively have a tapering shape that is not substantially symmetrical to its longitudinal axis A (as shown in figure 1) but is more adapted to the anatomy of the human ear canal.

Irrespective of the precise shape of the head portion 14, the head portion 14 is preferably dimensioned in such a way that it cannot be introduced into the inner part of the outer ear canal of the subject's outer ear. In the shown exemplary embodiment, the distal end 18 of the head portion 14 has a substantially round shape. Only a few millimeters (less than 4mm) away from the distal end point 34 in the direction of the longitudinal axis A, the head portion 14 exhibits a diameter of more than 5mm. Since the inner part of the outer ear canal of an adult usually exhibits a diameter of 4mm, there is no risk that the distal end 18 of the head portion 14 is inadvertently introduced too deeply into the subject's ear canal. Therefore, injuries to the sensitive skin of the inner part of the outer ear canal and/or to the eardrum can be reliably avoided.

The movable portion 20 comprises a first bore 36 extending substantially along the axial direction A of the head portion 14, but not exactly parallel thereto. The distal end of the first bore 36 is located in proximity to the distal end point 34, but is offset with its bore axis B by at least 2mm from the longitudinal axis A. Furthermore, the distal end of the first bore 36 is closed by a plate 38. An enlarged top view of the plate 38 is shown in figure 2. Since the bore 36 is cylindrical in shape, in figure 2 the plate 38 has a generally circular appearance with the bore axis B forming the center thereof. However, the bore 30 and/or the plate 38 may equally exhibit other shapes.

The plate 38 supports a wide-angle color video camera 40 and distal ends of four light guides 42. In the exemplary embodiment, the light guides 42 are located around the video camera 40, such that one light guide 42 is associated to each of the four lateral sides of the substantially rectangular video camera 40. However, this is not a prerequisite for the present device. Instead of four light guides 42, for example, only two light guides 42 may be provided in the ear inspection device 10. The video camera 40 is advantageously a wafer-level camera of dimensions in the 1 to 2mm range having a substantially flat configuration. The wafer-level camera advantageously exhibits dimensions of only about 1 mm x 1 mm providing a resolution of about 250 pixels x 250 pixels. The plate 38 has a diameter between 1.5mm and 2.0mm and the light guides 42 have a diameter of only about 0.2mm.

The video camera 40 is connected to a distal end of a cable (not shown). The cable, e.g. a ribbon cable, extends through the bore 36 and into the handle portion 12 of the ear inspection device 10. A distal end of the cable is connected to a logic unit 44, such as a microprocessor, which is schematically illustrated in figure 1. Similarly, the light guides 42 (not shown in figure 1) extend through the bore 36 and into the handle portion 12 of the ear inspection device 10. Proximal ends of the light guides 42 are connected to four LEDs 46, respectively. The LEDs 46 are positioned - like the logic unit 44 - within the handle portion 12 of the ear inspection device 10. The LEDs 46 can be individually switched on and off. Furthermore, the handle portion 12 preferably comprises a memory 48 for storing images captured by the video camera 40. The memory may be formed e.g. by a storage card slot and a corresponding storage card inserted in the slot. The handle portion 12 may further comprise a display (not shown) for displaying to the user the images taken by the camera 40. Additionally or alternatively, the handle portion 12 may comprise a cable connection port, such as an USB-port, and/or a wireless connection, such as Bluetooth®, WIFI® and/or an energy supply, such as a (rechargeable) battery. These additional (optional) components of the handle portion 12 are known e.g. from modern digital cameras or mobile phones.

The first embodiment of the inventive ear inspection device 10 further comprises a second bore 50 extending along the longitudinal axis A, i.e. the axis of the second bore substantially coincides with the longitudinal axis A. Consequently, in this embodiment, the distal end of the second bore 50 substantially coincides with the distal end point 34 of the head portion 14. The distal end of the second bore 50 is closed by a plate comprising an infrared sensor unit 52 (only schematically shown herein) configured for detecting infrared radiation from the subject's ear. The infrared sensor unit 52 is connected to a distal end of a cable (not shown). The cable, e.g. a ribbon cable, extends through the second bore 50 and into the handle portion 12 of the ear inspection device 10. A distal end of the cable is also connected to the logic unit 44.

The main viewing direction of the infrared sensor unit 52 substantially corresponds to the longitudinal axis A and, thus, is angled to the main viewing direction of the electronic imaging unit 40, substantially corresponding to the bore axis B of the first bore 36. The two main viewing directions A and B preferably intersect at a point at which the subject's eardrum is supposed to be when the ear inspection device is properly introduced into the subject's exterior ear canal. In view of the typical length of the inner part of the subject's exterior ear canal, the distance may be between 3mm and 20mm.

The ear inspection device 10 shown in figures 1 and 2 may be used e.g. to detect the subject's body core temperature (i.e. the temperature at the eardrum) and/or to visually inspect the condition of the eardrum by capturing images thereof, e.g. in order to determine the likelihood of an inflammation thereof. Preferably, data of the subject's ear is acquired (preferably simultaneously or in close temporal relationship) by both units, i.e. by the infrared sensor unit 52 and the electronic imaging unit 40, so that the data acquired by one unit can be used to verify the data acquired by the respective other unit. Performing such a plausibility check allows for avoiding misleading or wrong results. Thus, the reliability and accuracy of the corresponding measurement can be increased. In particular, it is possible to assure with high reliability that the ear inspection device 10 is correctly positioned or oriented with respect to the subject's ear so as to assure that the main viewing directions of the units 40, 52 are pointing onto the eardrum.

For acquiring data of a subject's inner part of the external ear canal, and in particular of a subject's eardrum, the distal end 18 of the head portion 14 has to be introduced into the subject's ear canal. Due to the shape of the head portion 14 there is no risk to insert the distal end 18 too deeply into the ear canal. That is, the shape and geometry of the distal end 18 do not allow too deeply introducing the distal end point 34 into the subject's inner part of the outer ear canal which is pain sensitive. Therefore, injuries to the skin of the inner part of the outer ear canal and/or the eardrum can be reliably avoided. The geometry and the technology of the inventive ear inspection device do not require deforming the subject's ear as with a classic ear inspection device, as described above. Consequently, the ear inspection device according to the present invention can also be securely applied by lay persons.

Even though the distal end 18 of the head portion 14 will not be inserted into the inner part of the outer ear canal, the ear inspection device according to the present invention, nevertheless, allows for capturing images from the inner part of the outer ear canal and the eardrum, due to the wide angle camera 40 being provided at the distal end 18 of the head portion 14. In order to improve the ability of the camera 40 to "see" the eardrum, the camera 40 is placed offset from the longitudinal axis A of the head portion 14. Furthermore, the main "viewing direction" of the camera 40, corresponding to the bore axis B, is angled with respect to the longitudinal axis A of the head portion 14. The bore axis B and the longitudinal axis A intersect at a point having a predetermined distance from the distal end point 34, wherein the predetermined distance corresponds to the typical length of a subject's inner part of the outer ear canal, so that the camera 40 is directed to the eardrum.

When the distal end 18 of the head portion is introduced into the subject's ear canal, it may happen that artifacts, such as earwax particles or hair, in front of the camera 40, e.g. adhering to the probe cover, partially or even fully obstruct the view onto the eardrum. Therefore, the motion mechanism 24 may turn the rotatable portion 20 of the head portion 14 about its axis of rotation R with respect to the remaining ear inspection device 10. For example, the motion mechanism 24 may rotate the rotatable portion 20 from an initial position by about 120° in clockwise direction, then from the initial position by about 120 in counter-clockwise direction, and finally return to the initial position. The camera 40 may capture one or more images from each of these equally spaced three positions. The logic unit 44 may identify different objects in the subject's ear by comparing the images received from the camera 40. In particular, the logic unit 44 may discriminate artifacts from the eardrum by determining their distance to the camera 40 according to the principle of stereoscopic viewing, as described in more detail above.

In order to further improve the identification process, more than one image may preferably be taken from each of the three positions of the camera 40, with different LEDs 46 switched on and off for each captured image. Illumination of the artifacts and the eardrum from different positions also assists to discriminate these objects, as described in more detail above.

Finally, a new image may be generated (preferably by the logic unit 44) in which the identified artifacts are eliminated, so as to clearly show the eardrum. The degree of reddishness of the eardrum can then be easily determined. The user may be provided with corresponding information, such as to see the physician because of the risk of otitis media, or not. Also, if the ear inspection device failed to detect the eardrum because of massive earwax in the subject's ear canal, corresponding information may be provided to the user. The user may then decide to visit a physician for having his or her ear canal cleaned. Also the data acquired by the infrared sensor element 52 can be advantageously taken into account by the logic unit 44 in order to inform the operator of the ear inspection device 10 correspondingly.

As mentioned above, in the first embodiment of the inventive ear inspection device 10 shown in figures 1 and 2, the main viewing direction of the infrared sensor unit 52 substantially coincides with the longitudinal axis A of the head portion 14. Therefore, the infrared sensor unit 52 is not able - in contrast to the electronic imaging unit 40 - to "look around the corner".

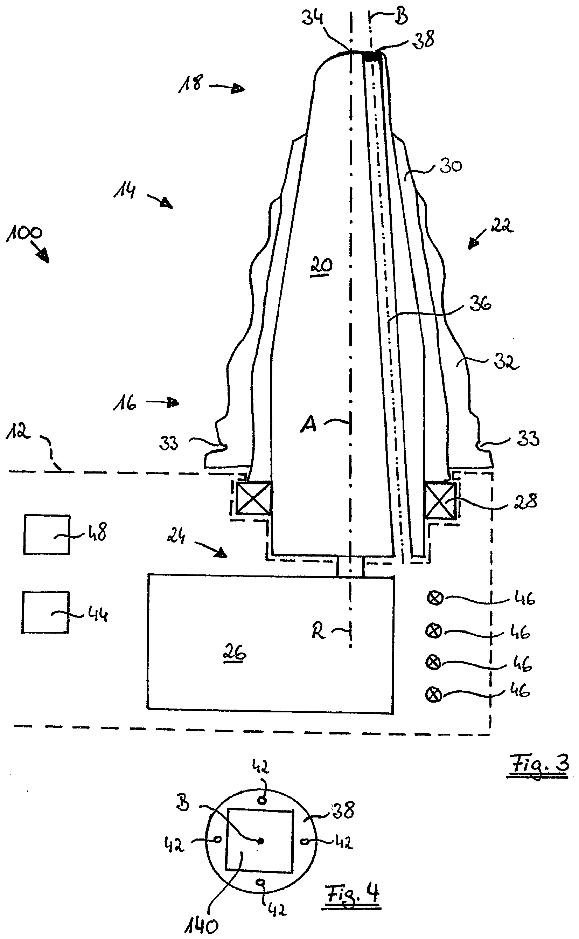

A further improved second embodiment of an inventive ear inspection device 100 is shown in figures 3 and 4. Like reference signs refer to the same parts as in the first embodiment. Therefore, a detailed description thereof is omitted here. The second embodiment differs from the first embodiment substantially only in that it does not comprise the second bore 50 with the cover plate having the infrared sensor unit 52. Instead, the infrared sensor unit 140 is formed integrally with the electronic imaging unit 140. That is, element 140 corresponds to a wafer-level camera capable of acquiring both, pictures of light in the visible range and pictures of light in the infrared range. Therefore, in this embodiment, both the electronic imaging unit and the infrared sensor unit are capable of "looking around the corner". Furthermore, the main viewing directions of both units coincide, as both units 140 are provided on the same chip.

## Claims

1. Ear inspection device (10; 100) configured for being at least partially introduced into a subject's external ear canal for determining a condition of the subject's ear, such as temperature, in particular at the subject's eardrum, wherein the ear inspection device (10; 100) comprises an infrared sensor unit (52; 140) configured for detecting infrared radiation from the subject's ear,
**characterized in that** the ear inspection device (10; 100) further comprises
- an electronic imaging unit (40; 140) configured for capturing images based on radiation in the visible range from the subject's ear;
- a handle portion (12) allowing a user to manipulate the ear inspection device (10; 100) during its application; and
- a head portion (14) exhibiting a substantially tapering form extending along a longitudinal axis (A) of the head portion (14);
wherein the head portion (14) has a proximal end (16) adjacent to the handle portion (12) and a smaller distal end (18) configured to be introduced in the subject's external ear canal;
wherein the electronic imaging unit (40; 140) is positioned at the distal end (18) of the head portion (14);
wherein the electronic imaging unit (40; 140) and/or the infrared sensor unit (52; 140) is/are positioned radially offset from the longitudinal axis (A) of the head portion (14); and wherein the ear inspection device (10; 100) further comprises a motion mechanism (24) configured to allow rotational displacement of the electronic imaging unit (40; 140) and/or the infrared sensor unit (140) around the longitudinal axis (A).

2. Ear inspection device (10; 100) according to claim 1, wherein the ear inspection device (10; 100) further comprises a logic unit (44) configured for receiving and processing signals from the infrared sensor unit (52; 140) and the electronic imaging unit (40; 140).

3. Ear inspection device (10; 100) according to claim 2, wherein the logic unit (44) is further configured for using the signals received from the electronic imaging unit (40; 140) and/or from the infrared sensor unit (52; 140) for verifying the correct positioning of the ear inspection device (10; 100) with respect to the subject's ear.

4. Ear inspection device (10; 100) according to claim 2 or 3, wherein the logic unit (44) is further configured for determining - based on the received signals from the electronic imaging unit (40; 140) - whether the ear inspection device (10; 100) is positioned within the left or the right ear of the subject.

5. Ear inspection device (10; 100) according to any one of claims 2 to 4, wherein the logic unit (44) is further configured for discriminating different objects in the subject's ear, such as earwax, hair and the eardrum, by comparing their appearance in at least two images captured by the electronic imaging unit (40; 140) from different positions within the ear canal and/or with illumination from different positions within the ear canal.

6. Ear inspection device (10; 100) according to any one of the preceding claims, wherein the infrared sensor unit (52; 140) comprises a plurality of infrared sensor elements for detecting infrared radiation from different regions of the ear.

7. Ear inspection device (100) according to any one of the preceding claims, wherein the infrared sensor unit (100) is formed by an infrared camera (140) configured for capturing images based on radiation in the infrared range from the subject's ear.

8. Ear inspection device (100) according to any one of the preceding claims, wherein the infrared sensor unit (140) is formed integrally with the electronic imaging unit (140).

9. Ear inspection device (10; 100) according to any one of the preceding claims, wherein the ear inspection device (10; 100) further comprises a fluid sensor unit adapted to detect fluid in the subject's ear.

10. Ear inspection device (10; 100) according to any one of the preceding claims, wherein the ear inspection device (10; 100) further comprises at least one light source also positioned at the distal end (18) of the head portion (14).

11. Method of determining a condition of a subject's ear, such as temperature, in particular at the subject's eardrum, wherein the method comprises the following steps:
- introducing an ear inspection device according to any one of the previous claims, at least partially into the subject's external ear canal, the ear inspection device (10; 100) comprising an infrared sensor unit (52; 140) and an electronic imaging unit (40; 140);
- detecting infrared radiation from the subject's ear using the infrared sensor (52; 140); and
- capturing at least one image based on radiation in the visible range from the subject's ear using the electronic imaging unit (40; 140).

12. Method according to claim 11 further comprising at least one of the following steps:
- verifying appropriate positioning of the ear inspection device (10; 100) with respect to the subject's ear based on the detected infrared radiation and/or the at least one captured image;
- determining whether the ear inspection device (10; 100) is positioned within the left or the right ear of the subject based on the at least one captured image; and
- discriminating different objects in the subject's ear, such as earwax, hair and the eardrum, by comparing their appearance in at least two images captured by the electronic imaging unit (40; 140) from different positions within the ear canal and/or with illumination from different positions within the ear canal.

## Patentansprüche

1. Ohruntersuchungsvorrichtung (10; 100), die dazu ausgebildet ist, zumindest teilweise in den Außenohr-Kanal einer Person eingeführt zu werden, um einen Zustand, wie zum Beispiel die Temperatur, des Ohrs der Person zu bestimmen, insbesondere am Trommelfell der Person, wobei die Ohruntersuchungsvorrichtung (10; 100) eine Infrarotsensoreinheit (52; 140) umfasst, die zur Detektion von Infrarotstrahlung vom Ohr der Person ausgebildet ist,
**dadurch gekennzeichnet, dass** die Ohruntersuchungsvorrichtung (10; 100) ferner umfasst
- eine elektronische Bildgebungseinheit (40; 140), die zur Erfassung von Bildern auf Grundlage von Strahlung vom Ohr der Person im sichtbaren Bereich ausgebildet ist;
- ein Griffabschnitt (12), der es dem Benutzer erlaubt, die Ohruntersuchungsvorrichtung (10; 100) während ihrer Anwendung zu manipulieren; und
- einen Kopfabschnitt (14), der eine sich im Wesentlichen verjüngende Form aufweist, die sich entlang einer Längsachse (A) des Kopfabschnitts (14) erstreckt;
wobei der Kopfabschnitt (14) angrenzend an den Griffabschnitt (12) ein proximales Ende (16) aufweist und ein kleineres distales Ende (18), das dazu ausgebildet ist, in den Außenohr-Kanal der Person eingeführt zu werden;
wobei die elektronische Bildgebungseinheit (40; 140) am distalen Ende (18) des Kopfabschnitts (14) angeordnet ist;
wobei die elektronische Bildgebungseinheit (40; 140) und/oder die Infrarotsensoreinheit (52; 140) radial versetzt von der Längsachse (A) des Kopfabschnitts (14) angeordnet ist/sind; und
wobei die Ohruntersuchungsvorrichtung (10; 100) ferner einen Bewegungsmechanismus (24) umfasst, der dazu ausgebildet ist, eine Dreh-Disklokalisation der elektronischen Bildgebungseinheit (40; 140) und/oder der Infrarotsensoreinheit (52; 140) um die Längsachse (A) zu erlauben.

2. Ohruntersuchungsvorrichtung (10; 100) nach Anspruch 1, wobei die Ohruntersuchungsvorrichtung (10; 100) ferner eine Logikeinheit (44) umfasst, die zum Empfangen und Verarbeiten von Signalen von der Infrarotsensoreinheit (52; 140) und der elektronischen Bildgebungseinheit (40; 140) ausgebildet ist.

3. Ohruntersuchungsvorrichtung (10; 100) nach Anspruch 2, wobei die Logikeinheit (44) ferner zur Verwendung von Signalen, empfangen von der elektronischen Bildgebungseinheit (40; 140) und/oder von der Infrarotsensoreinheit (52; 140), zur Verifizierung der richtigen Positionierung der Ohruntersuchungsvorrichtung (10; 100) hinsichtlich des Ohrs der Person ausgebildet ist.

4. Ohruntersuchungsvorrichtung (10; 100) nach Anspruch 2 oder 3, wobei die Logikeinheit (44) ferner dazu ausgebildet ist - auf Grundlage der von der elektronischen Bildgebungseinheit (40; 140) empfangenen Signale - zu bestimmen, ob die Ohruntersuchungsvorrichtung (10; 100) im linken oder rechten Ohr der Person positioniert ist.

5. Ohruntersuchungsvorrichtung (10; 100) nach einem der Ansprüche 2 bis 4, wobei die Logikeinheit (44) ferner dazu ausgebildet ist, zwischen verschiedenen Objekten im Ohr der Person, wie zum Beispiel Ohrenschmalz, Haaren und dem Trommelfell, mittels Vergleich ihres Erscheinungsbilds in mindestens zwei Bildern, die von der Bildgebungseinheit (40; 140) aus unterschiedlichen Positionen innerhalb des Ohrkanals und/oder mit Belichtung aus unterschiedlichen Positionen innerhalb des Ohrkanals erfasst wurden, zu unterscheiden.

6. Ohruntersuchungsvorrichtung (10; 100) nach einem der vorangegangenen Ansprüche, wobei die Infrarotsensoreinheit (52; 140) eine Mehrzahl von Infrarotsensorelementen zur Detektion von Infrarotstrahlung von verschiedenen Regionen des Ohrs umfasst.

7. Ohruntersuchungsvorrichtung (100) nach einem der vorangegangenen Ansprüche, wobei die Infrarotsensoreinheit (100) durch eine Infrarotkamera (140) gebildet wird, die zur Erfassung von Bildern auf Grundlage von Strahlung im Infrarotbereich vom Ohr der Person ausgebildet ist.

8. Ohruntersuchungsvorrichtung (100) nach einem der vorangegangenen Ansprüche, wobei die Infrarotsensoreinheit (140) integral mit der elektronische Bildgebungseinheit (140) gebildet ist.

9. Ohruntersuchungsvorrichtung (10; 100) nach einem der vorangegangenen Ansprüche, wobei die Ohruntersuchungsvorrichtung (10; 100) ferner eine Fluidsensoreinheit umfasst, die zur Detektion von Fluid im Ohr der Person ausgebildet ist.

10. Ohruntersuchungsvorrichtung (10; 100) nach einem der vorangegangenen Ansprüche, wobei die Ohruntersuchungsvorrichtung (10; 100) ferner zumindest eine Lichtquelle umfasst, die ebenfalls am distalen Ende (18) des Kopfabschnitts (14) angeordnet ist.

11. Verfahren zur Bestimmung eines Zustands eines Ohrs einer Person, wie zum Beispiel Temperatur, insbesondere am Trommelfell der Person, wobei das Verfahren die folgenden Schritte umfasst:
- Einführen einer Ohruntersuchungsvorrichtung nach einem der vorangegangenen Ansprüche zumindest teilweise in den Außenohr-Kanal einer Person, wobei die Ohruntersuchungsvorrichtung (10; 100) eine Infrarotsensoreinheit (52; 140) und eine elektronische Bildgebungseinheit (40; 140) umfasst;
- Detektieren von Infrarotstrahlung vom Ohr der Person unter Verwendung der Infrarotsensoreinheit (52; 140); und
- Erfassen wenigstens eines Bildes auf Grundlage von Strahlung im sichtbaren Bereich vom Ohr der Person unter Verwendung der elektronischen Bildgebungseinheit (40; 140).

12. Verfahren nach Anspruch 11 ferner umfassend wenigstens einen der folgenden Schritte:
- Verifizieren der richtigen Positionierung der Ohruntersuchungsvorrichtung (10; 100) hinsichtlich des Ohrs der Person auf Grundlage der detektierten Infrarotstrahlung und/oder des wenigstens einen erfassten Bildes;
- Bestimmen, ob die Ohruntersuchungsvorrichtung (10; 100) im rechten oder linken Ohr der Person positioniert ist, auf Grundlage des wenigstens einen erfassten Bildes; und
- Unterscheiden unterschiedlicher Objekte im Ohr der Person, wie zum Beispiel Ohrenschmalz, Haaren und dem Trommelfell, mittels Vergleich ihres Erscheinungsbilds in mindestens zwei Bildern, die von der Bildgebungseinheit (40; 140) aus unterschiedlichen Positionen innerhalb des Ohrkanals und/oder mit Belichtung aus unterschiedlichen Positionen innerhalb des Ohrkanals erfasst wurden.

## Revendications

1. Dispositif d'inspection d'oreille (10; 100) configuré pour être au moins partiellement introduit dans un canal d'oreille externe d'un sujet pour une détermination d'une condition de l'oreille du sujet, telle qu'une température, en particulier au niveau du tympan du sujet, dans lequel le dispositif d'inspection d'oreille (10 ; 100) comprend une unité de capteur infrarouge (52 ; 140) configurée pour une détection de rayonnement infrarouge à partir de l'oreille du sujet, **caractérisé en ce que** le dispositif d'inspection d'oreille (10 ; 100) comprend en outre
- une unité d'imagerie électronique (40 ; 140) configurée pour une capture d'images sur la base d'un rayonnement dans la plage visible à partir de l'oreille du sujet ;
- une portion poignée (12) permettant à un utilisateur de manipuler le dispositif d'inspection d'oreille (10 ; 100) pendant son application ; et
- une portion tête (14) présentant une forme sensiblement conique s'étendant le long d'un axe longitudinal (A) de la portion tête (14) ;
dans lequel la portion tête (14) a une extrémité proximale (16) adjacente à la portion poignée (12) et une extrémité distale (18) plus petite configurée pour être introduite dans le canal d'oreille externe du sujet ;
dans lequel l'unité d'imagerie électronique (40 ; 140) est positionnée au niveau de l'extrémité distale (18) de la portion tête (14) ;
dans lequel l'unité d'imagerie électronique (40 ; 140) et/ou l'unité de capteur infrarouge (52 ; 140) est/sont positionnée(s) radialement en décalage à partir de l'axe longitudinal (A) de la portion tête (14) ; et
dans lequel le dispositif d'inspection d'oreille (10 ; 100) comprend en outre un mécanisme de mouvement (24) configuré pour permettre un déplacement en rotation de l'unité d'imagerie électronique (40 ; 140) et/ou de l'unité de capteur infrarouge (140) autour de l'axe longitudinal (A).

2. Dispositif d'inspection d'oreille (10 ; 100) selon la revendication 1, dans lequel le dispositif d'inspection d'oreille (10 ; 100) comprend en outre une unité logique (44) configurée pour une réception et un traitement de signaux à partir de l'unité de capteur infrarouge (52 ; 140) et l'unité d'imagerie électronique (40 ; 140).

3. Dispositif d'inspection d'oreille (10 ; 100) selon la revendication 2, dans lequel l'unité logique (44) est en outre configurée pour une utilisation des signaux reçus à partir de l'unité d'imagerie électronique (40 ; 140) et/ou à partir de l'unité de capteur infrarouge (52 ; 140) pour une vérification du positionnement correct du dispositif d'inspection d'oreille (10 ; 100) par rapport à l'oreille du sujet.

4. Dispositif d'inspection d'oreille (10 ; 100) selon la revendication 2 ou 3, dans lequel l'unité logique (44) est en outre configurée pour une détermination, sur la base des signaux reçus à partir de l'unité d'imagerie électronique (40 ; 140), du fait que le dispositif d'inspection d'oreille (10 ; 100) est positionné à l'intérieur de l'oreille gauche ou droite du sujet.

5. Dispositif d'inspection d'oreille (10 ; 100) selon l'une quelconque des revendications 2 à 4, dans lequel l'unité logique (44) est en outre configurée pour une distinction de différents objets dans l'oreille du sujet, tels que du cérumen, des poils et le tympan, en comparant leur apparence dans au moins deux images capturées par l'unité d'imagerie électronique (40 ; 140) à partir de différentes positions à l'intérieur du canal d'oreille et/ou avec une illumination à partir de différentes positions à l'intérieur du canal d'oreille.

6. Dispositif d'inspection d'oreille (10 ; 100) selon l'une quelconque des revendications précédentes, dans lequel l'unité de capteur infrarouge (52 ; 140) comprend une pluralité d'éléments de capteur infrarouge pour une détection de rayonnement infrarouge à partir de différentes régions de l'oreille.

7. Dispositif d'inspection d'oreille (100) selon l'une quelconque des revendications précédentes, dans lequel l'unité de capteur infrarouge (100) est formée par une caméra infrarouge (140) configurée pour une capture d'images sur la base d'un rayonnement dans la plage infrarouge à partir de l'oreille du sujet.

8. Dispositif d'inspection d'oreille (100) selon l'une quelconque des revendications précédentes, dans lequel l'unité de capteur infrarouge (140) est formée intégralement avec l'unité d'imagerie électronique (140).

9. Dispositif d'inspection d'oreille (10 ; 100) selon l'une quelconque des revendications précédentes, dans lequel le dispositif d'inspection d'oreille (10 ; 100) comprend en outre une unité de capteur de fluide adaptée pour une détection d'un fluide dans l'oreille du sujet.

10. Dispositif d'inspection d'oreille (10 ; 100) selon l'une quelconque des revendications précédentes, dans lequel le dispositif d'inspection d'oreille (10 ; 100) comprend en outre au moins une source de lumière également positionnée au niveau de l'extrémité distale (18) de la portion tête (14).

11. Procédé de détermination d'une condition d'une oreille d'un sujet, telle qu'une température, en particulier au niveau du tympan du sujet, dans lequel le procédé comprend les étapes suivantes :
- une introduction d'un dispositif d'inspection d'oreille selon l'une quelconque des revendications précédentes, au moins partiellement dans le canal d'oreille externe du sujet, le dispositif d'inspection d'oreille (10 ; 100) comprenant une unité de capteur infrarouge (52 ; 140) et une unité d'imagerie électronique (40 ; 140) ;
- une détection de rayonnement infrarouge à partir de l'oreille du sujet en utilisant le capteur infrarouge (52 ; 140) ; et
- une capture d'au moins une image sur la base d'un rayonnement dans la plage visible à partir de l'oreille du sujet en utilisant l'unité d'imagerie électronique (40 ; 140).

12. Procédé selon la revendication 11 comprenant en outre au moins une des étapes suivantes :
- une vérification d'un positionnement approprié du dispositif d'inspection d'oreille (10 ; 100) par rapport à l'oreille du sujet sur la base du rayonnement infrarouge détecté et/ou de l'au moins une image capturée ;
- une détermination du fait que le dispositif d'inspection d'oreille (10 ; 100) est positionné à l'intérieur de l'oreille gauche ou droite du sujet sur la base de l'au moins une image capturée ; et
- une distinction de différents objets dans l'oreille du sujet, tels que du cérumen, des poils et le tympan, en comparant leur apparence dans au moins deux images capturées par l'unité d'imagerie électronique (40 ; 140) à partir de différentes positions à l'intérieur du canal d'oreille et/ou avec une illumination à partir de différentes positions à l'intérieur du canal d'oreille.
